Europäisches Patentamt

(19) European Patent Office   (11) Publication number:   **0 002 003**

Office européen des brevets                                          **B1**

(12)                    EUROPEAN PATENT SPECIFICATION

(45) Date of publication of patent specification: **05.09.84**   (51) Int. Cl.³: **A 61 L 15/01,** A 61 K 33/08,
                                                                   A 61 K 7/00
(21) Application number: **78101282.8**

(22) Date of filing: **02.11.78**

(54) **Medical and cosmetic compositions containing a thixotropic aluminium derivative; process for producing them.**

㉚ Priorrity: **12.11.77 GB 4717077**

(43) Date of publication of application:
**30.05.79 Bulletin 79/11**

(45) Publication of the grant of the patent:
**05.09.84 Bulletin 84/36**

(84) Designated Contracting States:
**BE CH DE FR LU NL SE**

(56) References cited:
**FR-A- 738 051**
**FR-A- 881 367**
**FR-A-2 274 278**
**GB-A- 572 142**
**GB-A- 784 706**
**GB-A-1 335 358**
**US-A-3 873 686**

The file contains technical information
submitted after the application was filed and
not included in this specification

(73) Proprietor: **Kuy, Bert**
**Hotzestrasse 28**
**CH-8006 Zürich (CH)**

(72) Inventor: **Kuy, Bert**
**Hotzestrasse 28**
**CH-8006 Zürich (CH)**

(74) Representative: **Kraus, Walter, Dr. et al**
**Patentanwälte Kraus & Weisert Irmgardstrasse**
**15**
**D-8000 München 71 (DE)**

Courier Press, Leamington Spa, England.

## Description

Field of the Invention

The present invention relates to novel composition of matter, and a composite material comprising it for use in medicine and cosmetics. The novel composition and composite material are adapted to provide effective healing and soothing effects in the form of bandages, such as hot and cold bandages.

The novel composition and composite material is characterized by healing and soothing effects when applied externally to afflicted areas of the skin.

This invention relates to a composition for use in medicine and for use in cosmetics comprising as active ingredient an aqueous thixotropic gel of about 3 to 20 per cent by weight of a microcrystalline aluminum hydroxy chloride having an atomic ratio of aluminum to chlorine of from 5 to 1 to 6 to 1.

The active ingredient of the compositions according to the present invention is a colloidal system of an aluminum chloride oxide hydrate having an atomic ratio of aluminum to chlorine in the range of from 5 to 1 to 6 to 1 and this can be presented by the general formula $Al_x(OH)_yCl_z$ wherein x, y and z are as required for the respective valencies of aluminum, chlorine and the hydroxy groups. It is believed that the more exact formula of the active ingredients is $[Al(OH)_3]_xAl(OH)_2Cl$ wherein x varies between 4 and 5. In the following the active material is designated as "aluminum hydroxide chloride".

The pH of the thixotropic composition varies between about 3.5 and 5, and generally between 3.5 and 3.8.

The novel composition may optionally contain additional ingredients according to the specific uses of the said compositions. Said additional ingredients may be, for example, fatty substances, such as lanolin, collagenase, polyalkylene, polyethyleneglycol and organic acids, like tartaric acid, propionic acid, citric and lactic acid, disinfectants, bacteriostats, bactericides and antimycotics, germicidal, fungicidal and fungistatic substances and glycerol.

The said thixotropic aluminum hydroxy chloride is advantageously used to imbue a suitable inert carrier, such as a cellulosic material (paper, cellulose, cotton wool, cotton, etc.) or any other suitable carrier such as foams, fabrics, mixed fabrics, non-woven fabrics and gauzes.

A suitable quantity of the active ingredient is used to imbue or impregnate the said carrier. According to one embodiment the thixotropic gel is agitated so as to reduce its viscosity and then it is applied to the porous, pliable, polymeric carrier. This carrier is impregnated with a predetermined quantity of said thixotropic gel, for instance, by squeezing the thixotropic solution into the carrier, the quantity of the active ingredient being such as to provide as high an effective quantity of the active material as can be supported by said carrier. Representative values of such quantities are from 10 to 35 g per 100 cm² of a material such as plastic foam or gauze.

The active ingredient, i.e. the aluminum hydroxide chloride exerts an astringent action on human skin, without causing undue irritation. It exerts a certain acid binding activity, and due to its inherent pH of about 3.5 to pH 7 it is well tolerated by human skin. When admixed with a suitable quantity of water, there is obtained a thixotropic system into which there are readily incorporated substances such as lanolin. The latter is generally obtained as stable system with aqueous ingredients only in conjunction with other constituents such as surface active agents or the like. The gel exerts an antiphlogistic and soothing action.

The active ingredient is provided in the form of a microcrystalline aluminum hydroxide in which part of the hydroxy groups have been replaced by chlorine, to give the above defined composition with the stipulated molar ratio of aluminum to chlorine. The substance is thixotropic. About 10 per cent by weight of the said hydroxy-chloride in water forms a gel which can be easily liquefied by application of mechanical forces, such as by shaking, brushing or the like. The viscosity decreases thereby about 100 fold, from values in the range of 10,000 cps to about 100 cps. When left to stand it reverts again to the high viscosity.

The thixotropic substance can be applied directly to the skin or mucosa for medical uses. It can be provided in conjunction with bandages which serve as carrier of the said active ingredient. The water of the said composition evaporates in a gradual matter, and there remains a coating of aluminum-hydroxide-chloride, which allows the skin to breathe and which maintains its activity over a prolonged period of time.

The new composition or composite material are generally used externally and thus very little of the essentially insoluble material is absorbed, if any at all. Toxicity tests indicate that the application of oral dosages of about 5 g/kg weight of the thixotropic aluminum hydroxy chloride to rats does not result in toxic effects.

The bandage may be backed with a layer such as aluminum foil and can be used as hot compress of prolonged useful duration of application.

When the active ingredient is used in conjunction with a suitable carrier, the said carrier is imbued with the active ingredient either by pressure; under reduced pressure, by brushing, coating or spraying. There is obtained a substantially homogenous product carrying a predetermined quantity of active material per unit area.

The novel composition can be mechanically applied by rolling, brushing and spraying but also by dipping in any quantity required on or in the suitable carrier.

The novel composition can be distributed easily in minimum or maximum quantities in an equal and homogenous manner without flowing off and has a strong adhesion on porous, homogenous or lipoid surfaces of materials like textiles, synthetic and natural materials.

For addition of further additives to the novel composition there is no need to use an emulsifier.

The novel composition is stable over a long period of time. It can be brought into any consistency required by applying pressure or any mechanical forces like vacuum. This is very important for its economic use in medicine and cosmetics.

The new composition is reversible in binding high volumes of moisture or water. It is insoluble in water and is washed off with difficulty.

### Example 1

A thixotropic substance is prepared from colloidal aluminum-chloride containing about 5 to 1 to about 6:1 atoms of aluminum per atom of chlorine in a concentration of about 10 weight per cent in water. The pH of the substance is about 4.0 and when at rest the viscosity is about 10.000 cps. Upon vigorous shaking the viscosity goes down to about 1.00 cps.

### Example 2

A thixotropic composition was prepared by incorporating 5 grams of lanolin into a composition of Example 1. The lanolin was gradually introduced with vigorous agitation and there was added a quantity of about 0.3 g potassium chloride per 100 mg of the thixotropic substance. A uniform, stable composition was obtained.

### Example 3

A thixotropic substance was prepared by incorporating about 1.0% of micronized silver sulfadiazine into a composition of Example 2. There was obtained a thixotropic substance of special value in the treatment of infected wounds.

### Example 4

A thixotropic substance was prepared by incorporating 1 to 3 per cent by weight of PVP-iodine into a composition of Example 1. A germicidal substance was obtained.

### Example 5

A thixotropic substance was prepared by incorporating a quantity of from 1 to 5 per cent of alginate into a composition of Example 1.

### Example 6

A composition prepared according to Example 1 was incorporated into up to about 70% by weight of pulverized polymeric foam (such as polyurethane powder). There was obtained a kneadable material which solidifies under refrigeration or upon evaporation of the larger part of its water content.

### Example 7

A highly porous polymeric foam (polyurethane foam) in ribbon form was imbued by brushing with liquefied material prepared according to Example 1. The quantity used was about 30 grams of the said substances per 100 cm² of the said carrier. By application of pressure on the foam carrying the thixotropic material, a substantially uniform distribution of the said active ingredient throughout the carrier was obtained, when the pressure was released. This foam can be refrigerated and used for the effective application of cold during a prolonged period of time.

### Example 8

A polyurethane ribbon of the type used in Example 7 was placed on a thin non-woven fabric and impregnated as set out in Example 7 by applying the thixotropic material from the open side of the foam. The product constitutes a suitable carrier for application on certain types of wounds.

### Example 9

Gauze was impregnated so as to provide about 20 grams of the substance of Example 1 per 100 gram of the gauze. The impregnation was effected for example by brushing.

### Example 10

Non woven cellulose fabric was imbued in a manner set out in Example 8. The quantity used was 10 gram of the material of Example 1 per 100 gram of the fabric.

### Example 11

A facial mask was made from a substance according to Example 6 and into which there was incorporated a small quantity of avocado juice. The mask was applied to the face in the form of a kneadable composition according to Example 6 and left in place in the form of a layer of about 4 mm during 20 minutes. Most of the fatty deposits were removed, redness of the skin decreased substantially and the skin was tightened. The skin was thereby cleaned and refreshed.

### Example 12

A composition of matter according to Example 1 was applied by means of cotton wads to the scalp and there was obtained fluffy good looking hair and the fat glands were cleaned. The shiny appearance of the skin disappeared.

### Example 13

An impregnated ribbon of Example 7 was applied to edemas and various wounds after surgery, wounds after accidents and burns of up to the third degree. There was experienced a pronounced effect of stimulation of granulation and acceleration of smooth epithelization without irritation. Pains were allayed and itching was reduced. A marked anti-inflammatory effect was experienced and the healing of the wounds took place speedily and without contamination by bacteria or fungi as bacteria and fungi cannot grow on a surface coated with the active material of the present invention. The bandage does not adhere to the wound and can be readily removed when so desired, without damage to the underlying tissues.

The rim of the wound is maintained in a soft state and the prevention of the hardening of the wound rim is one of the main effects of the novel compositions. When the bandage is removed, there do not remain any noxious or detrimental constituents which have to be removed.

The novel compositions were tested with a number of patients. Amongst these was a patient with an open extensive wound of the surface of the abdomen, of about 40 cm times 5 cm. This wound did not heal during 2 years. The bandage was applied, changed from time to time (70 times, twice daily). At the end of the period of 35 days the wound was clean, inflammation disappeared, a smooth epithelization had taken place and this made possible transplantations of skin and the healing of the residual surface. The patient was released from hospital after this treatment and the wound healed completely.

A patient 90 years old with spastic right arm with decubitis for about 2 years under the armpit, with no possibility of effective treatment by conventional means was treated with a cooled bandage according to Example 10. This was changed daily during one week. After this period of time a complete healing was attained.

A patient with second degree burns on both legs was treated by the application of bandages of Example 10. These were changed thrice daily. After 6 days the wound was clean and made possible a skin transplantation.

### Example 14

A patient with fractures of a leg was treated by the application of bandages according to Example 7 which were refrigerated prior to application. The bandages were changed every 30 minutes and after 4 hours the leg was free of oedemas and was ready for surgery.

### Example 15

A thixotropic aluminum hydroxy chloride according to Example 1 was used for impregnation of a non-woven fabric or special absorbent paper. The quantity applied was 3 g per unit and there were packed in individual packages of 5.08 × 7.62 cm (2½ × 3") envelopes, which were substantially hermetic. According to a preferred embodiment some perfume is added to provide a refreshening effect.

### Example 16

A composition of matter according to Example 7 was used for intime care. There was obtained a refreshening effect and an antimycotic activity. The deposit on the mucosa resulted in a perceptible effect during 4 hours.

### Example 17

A composition of matter was prepared consisting of 60 parts by weight of a substance according to Example 1 with 40 parts by weight of polyurethane foam in dust form. From this substance suppositories were formed which were used for the treatment of haemerrhoids. The suppositories were frozen and applied in this form. The slow melting brings about a uniform distribution of the active ingredient and this results in relief and in an improved effect.

### Example 18

An impregnated ribbon according to Example 7 was used in frozen form for cryotherapy. This can be effectively used for the treatment of muscle spasms, of swellings and inflammations of the musculo-sceletal system, for the treatment of post-surgical edemas, for the treatment of phlebitis and of rheumatoid arthritis.

A patient with myositis of the left gastrocnemius muscle was treated with such an ice-bandage, the duration of each application being 15 minutes. After the initial analgesia, mobilization exercises were started and continued for ten minutes. After this the bandage was removed and the procedure was repeated with a fresh bandage. The therapeutic results were obtained much quicker than with conventional methods of treatment.

### Example 19

Non-woven cotton was mixed with a composition according to Example 1. After initial drying an insole form was cut and this was applied to the bottom surface of the foot. A stocking was worn by the person, who had a history of excessive sweating (hyperhydrosis). After one day of regular activity, the foot with this insole was free of sweat, while the other foot was wet. This insole also prevented the development of fungi.

Another test was carried out with an insole of polyurethane foam impregnated by a composition according to Example 1. This was used for application under the armpit. After one day the area was dry. The other armpit was wet and an acrid smell was perceptible.

### Claims

1. A composition for use in medicine and for use in cosmetics comprising as active ingredient an aqueous thixotropic gel of about 3 to 20 per cent by weight of microcrystalline aluminium hydroxy chloride having an atomic ratio of aluminium to chlorine of from 5 to 1 to 6 to 1.

2. A composition according to claim 1, containing a quantity of a fatty substance, collagenase, polyalkylene, polyethyleneglycol, organic acid, of a germicidal, fungicidal, bacteriostatic or fungistatic substance or glycerol.

3. A composition according to claim 2, containing as organic acid tartaric, propionic, citric or lactic acid.

4. A composite material for use in medicine for topical application, which comprises a suitable carrier supporting as active ingredient a composition according to claim 1, 2 or 3.

5. A composite material according to claim 4, wherein the carrier is a bandage.

6. A composite material according to claim 4 or 5, wherein the carrier is a porous polymeric foam.

7. A composite material according to claim 6, wherein the foam is open-pore polyurethane foam.

8. A composite material according to claim 4 or 6, wherein the carrier is a cellulosic material.

9. A composite material according to claim 4, wherein the carrier is paper, gauze, cotton-wool, non-woven cellulosic fabric or felt.

10. A composite material for use in medicine in the form of absorbent pads containing an effective quantity of an active ingredient according to claim 1, 2 or 3.

## Patentansprüche

1. Zusammensetzung, die auf medizinischem und kosmetischem Gebiet verwendet werden kann, dadurch gekennzeichnet, daß sie als aktiven Bestandteil ein wäßriges thixotropes Gel aus etwa 3 bis 20 Gew.-% mikrokristallinem Aluminiumhydroxychlorid mit einem Atomverhältnis von Aluminium zu Chlor von 5:1 bis 6:1 enthält.

2. Zusammensetzung nach Anspruch 1, dadurch gekennzeichnet, daß sie eine fettartige Substanz, Collagenase, Polyalkylen, Polyethylenglykol, eine organische Säure oder eine germizide, fungizide, bakteriostatische oder fungistatische Substanz oder Glycerin enthält.

3. Zusammensetzung nach Anspruch 2, dadurch gekennzeichnet, daß sie als organische Säure Weinsäure, Propionsäure, Zitronensäure oder Milchsäure enthält.

4. Verbundmaterial für die Verwendung auf medizinischem Gebiet und die topische Anwendung, dadurch gekennzeichnet, daß es einen geeigneten Träger enthält, der als aktiven Bestandteil eine Zusammensetzung nach einem der Ansprüche 1, 2 oder 3 enthält.

5. Verbundmaterial nach Anspruch 4, dadurch gekennzeichnet, daß der Träger in Form einer Bandage vorliegt.

6. Verbundmaterial nach Anspruch 4 oder 5, dadurch gekennzeichnet, daß der Träger poröser polymerer Schaum ist.

7. Verbundmaterial nach Anspruch 6, dadurch gekennzeichnet, daß der Schaum ein offenporiger Polyurethanschaum ist.

8. Verbundmaterial nach Anspruch 4 oder 6, dadurch gekennzeichnet, daß der Träger ein cellulosehaltiges Material ist.

9. Verbundmaterial nach Anspruch 4, dadurch gekennzeichnet, daß der Träger Papier, Gaze, Baumwollwolle, nichtgewebtes cellulosehaltiges Flächenmaterial oder Filz ist.

10. Verbundmaterial für die Verwendung auf medizinischem Gebiet in Form von absorbierenden Tampons bzw. Kissen, dadurch gekennzeichnet, daß es eine wirksame Menge eines aktiven Bestandteils nach einem der Ansprüche 1, 2 oder 3 enthält.

## Revendications

1. Composition utilisable en médecine et dans des cosmétiques, caractérisée en ce qu'elle comprend à titre d'ingrédient actif, un gel tixotrope aqueux d'environ 3 à 20 % en poids d'hydroxychlorure d'aluminium microcristallin ayant un rapport atomique d'aluminium à chlore allant de 5:1 à 6:1.

2. Composition selon la revendication 1, caractérisée en ce qu'elle contient une quantité d'une substance grasse, de collagénase, de polyalkylène, de polyéthylèneglycol, d'acide organique, d'une substance germicide, fongicide, bactériostatique ou fongistatique ou de glycérol.

3. Composition selon la revendication 2, caractérisée en ce qu'elle contient en tant qu'acide organique de l'acide tartrique, propionique, citrique ou lactique.

4. Matière composite utilisable en médecine pour une application topique, caractérisée en ce qu'elle comprend un support convenable portant à titre d'ingrédient actif, une composition selon la revendication 1, 2 ou 3.

5. Matière composite selon la revendication 4, caractérisée en ce que le support est un pansement.

6. Matière composite selon la revendication 4 ou 5, caractérisée en ce que le support est une mousse polymère poreuse.

7. Matière composite selon la revendication 6, caractérisée en ce que la mousse est une mousse de polyuréthane à pores ouverts.

8. Matière composite selon la revendication 4 ou 6, caractérisée en ce que le support est un matériau cellulosique.

9. Matière composite selon la revendication 4, caractérisée en ce que le support est du papier, de la gaze, de l'ouate de coton, un tissue cellulosique non tissé ou du feutre.

10. Matière composite utilisable en médecine, caractérisée en ce qu'elle se trouve sous forme de tampons absorbants, contenant une quantité efficace d'un ingrédient actif selon l'une des revendications 1, 2 ou 3.